# EUROPÄISCHE PATENTSCHRIFT

(11) **EP 3 899 564 B1**
(45) Veröffentlichungstag und Bekanntmachung des Hinweises auf die Patenterteilung: **25.09.2024**
(21) Anmeldenummer: 19832608.4
(22) Anmeldetag: 16.12.2019
(51) Int. Cl.: G01R 33/38, A61B 5/055, G01R 33/381, G01R 33/383, G01R 33/46, G01R 33/36

(54) **NMR-MESSGERÄT MIT EINER SENSORVORRICHTUNG ZUR INTRAKORPORALEN ANORDNUNG**
NMR MEASURING APPARATUS HAVING A SENSOR DEVICE FOR INTRACORPOREAL ARRANGEMENT
APPAREIL DE MESURE À RÉSONANCE MAGNÉTIQUE NUCLÉAIRE DOTÉ D'UN DISPOSITIF CAPTEUR DESTINÉ À ÊTRE DISPOSÉ À L'INTÉRIEUR DU CORPS

(30) Priorität: 20.12.2018 DE 102018222446
(43) Veröffentlichungstag der Anmeldung: 27.10.2021
(73) Patentinhaber: Robert Bosch GmbH, 70442 Stuttgart (DE)
(72) Erfinder: HOFFMANN, Jens, 72070 Tuebingen (DE); GRAESSL, Andreas, 70771 Leinfelden-Echterdingen (DE); SCHLAWNE, Carolin, 70563 Stuttgart (DE)
(86) Internationale Anmeldenummer: PCT/EP2019/085323
(87) Internationale Veröffentlichungsnummer: WO 2020/127028

(56) Entgegenhaltungen:
- EP-A1- 3 185 028
- DE-T2- 60 017 072
- DE-T2- 60 017 074
- US-A- 5 072 732
- US-A1- 2012 223 705

## Beschreibung

### Stand der Technik

Die vorliegende Erfindung betrifft ein NMR- Messgerät, umfassend zumindest eine Sensorvorrichtung zur intrakorporalen Anordnung in einem menschlichen oder tierischen Körper, wobei die Sensorvorrichtung zumindest eine Magnet- Vorrichtung zur Erzeugung eines homogenen Magnetfelds BO, eine induktive Detektions-Vorrichtung zum Senden eines Anregungssignals B1 und zum Empfangen eines Kernspinresonanzsignals, und eine anwendungsspezifische elektrische Schaltung zur Vorprozessierung von mittels der induktiven Detektions-Vorrichtung empfangenen Kernspinresonanzsignalen aufweist. Aus den Dokumenten US 2012/0223705 A1, DE 600 17 074 T2, DE 600 17 072 T2 und US 5,072,732 A sind jeweils implantierbare NMR-Messgeräte bekannt. Das Dokument EP 3 185 028 A1 beschreibt zudem ein handgehaltenes NMR-Messgerät, welches zur Durchführung von Messungen in Körpernähe verbracht wird.

### Offenbarung der Erfindung

Das erfindungsgemäße NMR-Messgerät nach Anspruch 1 umfasst zumindest eine zu einer intrakorporalen Anordnung in einem menschlichen oder tierischen Körper speziell eingerichtete Sensorvorrichtung und umfassend zumindest einen nicht biokompatiblen und nicht biofunktionalen extrakorporalen Teil, wobei die Sensorvorrichtung zumindest aufweist:
- eine Magnet-Vorrichtung zur Erzeugung eines homogenen Magnetfelds B0,
- eine induktive Detektions-Vorrichtung zum Senden eines Kernspinresonanz-Anregungssignals B1 und zum Empfangen eines Kernspinresonanzsignals, wozu die induktive Detektions-Vorrichtung zumindest eine RF-Spule aufweist,
- eine anwendungsspezifische elektrische Schaltung zumindest zur Vorprozessierung von mittels der induktiven Detektions-Vorrichtung empfangenen Kernspinresonanzsignalen, und
- ein biokompatibles und biofunktionales Gehäuse.

Erfindungsgemäß ist das NMR-Messgerät zur intrakorporalen Messung von NMR-Spektren mittels der Sensorvorrichtung eingerichtet.

Darüber hinaus umfasst das erfindungsgemäße NMR-Messgerät zumindest einen nicht biokompatiblen und nicht biofunktionalen anderen Teil, welcher zur extrakorporalen Anordnung eingerichtet ist, wobei der andere Teil einen Signalgenerator mit einer Schaltung zu einer Erzeugung des Anregungssignals aufweist,wobei die induktive Detektions-Vorrichtung der Sensorvorrichtung induktiv mit dem Signalgenerator des NMR-Messgeräts zur Einkopplung des Anregungssignals koppelbar ist, wenn dieser extrakorporal angeordnet ist, und wobei das NMR-Messgerät zur induktiven Kopplung geeignete Mittel aufweist, mit denen das von dem extrakorporalen Signalgenerator erzeugte Anregungssignal drahtlos in einen intrakorporal angeordneten und mittels zumindest der RF-Spule gebildeten Schwingkreis der induktiven Detektions-Vorrichtung einkoppelbar ist.

Weitere extrakorporale Komponenten können beispielsweise eine extrakorporal angeordnete Steuervorrichtung, eine extrakorporal angeordnete Auswertevorrichtung, eine extrakorporal angeordnete Ausgabevorrichtung, eine extrakorporal angeordnete Eingabevorrichtung, eine extrakorporal angeordnete Energieversorgungsvorrichtung oder dergleichen umfassen. Es wird vorgeschlagen, dass derartige extrakorporale Komponenten des NMR-Messgeräts - sofern sie in einer Ausführungsform des NMR-Messgeräts vorhanden sind - zumindest teilweise in einem Gehäuse eines extrakorporalen Teils des NMR-Messgeräts untergebracht sind, beispielsweise in einem handgehaltenen Auslesegerät.

Unter "vorgesehen" soll insbesondere speziell "programmiert", "ausgelegt" und/oder "ausgestattet" verstanden werden. Darunter, dass ein Objekt zu einer bestimmten Funktion "vorgesehen" ist, soll insbesondere verstanden werden, dass das Objekt diese bestimmte Funktion in zumindest einem Anwendungs- und/oder Betriebszustand erfüllt und/oder ausführt oder dazu ausgelegt ist, die Funktion zu erfüllen.

Das NMR-Messgerät umfasst zumindest die Sensorvorrichtung, die zur intrakorporalen Anordnung in einem menschlichen oder tierischen Körper vorgesehen und speziell eingerichtet ist. Die Sensorvorrichtung stellt eine spezielle, besonders kleinbauende und funktional gezielt ausgelegte Ausführungsform eines Kernspinresonanz-Sensors dar, der zumindest zur Detektion von Kernspinresonanzsignalen eines Bestandteils eines menschlichen oder tierischen Körpers, d.h. insbesondere von in dem Körper vorhandenen Stoffen wie Gewebe, Muskel, Blut, Interstitium oder dergleichen, vorgesehen ist. Die Sensorvorrichtung erlaubt es, Kernspinresonanzsignale zu messen, mittels der die Durchführung einer NMR-Spektralanalyse möglich ist. Unter "Bestandteil eines menschlichen oder tierischen Körpers" soll jeglicher materielle Teil des Körpers im biologischen Sinne verstanden werden (im Unterschied zu rein geometrischen oder physikalischen Definitionen), d.h. jeder materielle Teil des Körpers als Organismus, unabhängig davon, ob er lebt oder nicht. Somit stellt der Bestandteil eines menschlichen oder tierischen Körpers ein stoffliches Gebilde dar, das prinzipiell in unterschiedlichem Zusammenhang mit dem menschlichen oder tierischen Körper stehen kann. Beispielsweise kann der Bestandteil in anatomischem Zusammenhang mit der Struktur und/oder dem Aufbau des menschlichen oder tierischen Körpers stehen oder in physiologischem Zusammenhang mit den Körperfunktionen und den dabei innerhalb eines Körpers ablaufenden Stoffwechselprozessen. Ferner kann der Bestandteil auch im Zusammenhang mit biochemischen Prozessen auf mikrobiologischer Ebene stehen. Der Begriff "Bestandteil eines menschlichen oder tierischen Körpers" umfasst dabei sowohl flüssige als auch feste Bestandteile. Beispiele eines derartig definierten Bestandteils eines menschlichen oder tierischen Körpers sind Arme, Beine, Kopf (Körperteile), Haut, Gewebeproben, Nagel, Knochen, Gehirn, Körperflüssigkeiten wie Speichel, Blut und/oder Urin, Fäzes, Eiter, Furunkel, Abszesse oder dergleichen. Insbesondere lassen sich bei geeigneter Auswertung der Kernspinresonanzsignale Informationen hinsichtlich in dem Bestandteil vorhandener Substanzen und deren Konzentration ermitteln. Derart kann unter Verwendung des Messgeräts beispielsweise Blut und/oder Urin auf Entzündungsindikatoren, Krebsindikatoren, Nierenwerte, Mineralwerte, Drogen-Indikatoren, Blutzuckergehalt, Sauerstoffgehalt, Cholesteringehalt, Konzentrationen von Lipoprotein niedriger/hoher Dichte (LDL, HDL) oder andere Analyten untersucht werden.

Die Funktionsweise der Sensorvorrichtung als Kernspinresonanz-Sensor basiert auf dem kernphysikalischen Effekt, bei dem Atomkerne einer Materialprobe, hier des intrakorporalen Materials wie Blut, Gewebe, Interstitium oder dergleichen, in einem Magnetfeld (Magnetfeld B0) elektromagnetische Wechselfelder absorbieren (Anregungssignal B1) und emittieren (Kernspinresonanzsignal). Dabei beruht die Kernspinresonanz auf der Präzession (Larmorpräzession) von Kernspins der Atomkerne in der untersuchten Materialprobe um die Magnetfeldlinien eines konstanten, insbesondere statischen, ersten Magnetfelds B0. Insbesondere werden die Kernspins der Atomkerne in der Materialprobe durch das erste Magnetfeld B0 ausgerichtet. Wird Energie in Form eines elektromagnetischen Anregungssignals B1, insbesondere eines elektromagnetischen Wechselfeldes, beispielsweise eines gepulsten Magnetfeldes, auf die Atomkerne eingestrahlt, die mit der Larmorpräzession deren Kernspins in Resonanz ist (Energiequanten), so können die Atomkerne die Orientierung ihrer Spins relativ zum Magnetfeld B0 durch Absorption dieser Energie ändern. Das eingestrahlte Anregungssignal B1 dient daher der Anregung der Kernspins, die unter Energieaufnahme ihre Kernspinzustände ändern. Äquivalent führt die Emission von Energiequanten in Folge einer Rückkehr der angeregten Kernspins in ein anderes, niedrigeres Energieniveau, zur Emission eines elektromagnetischen Wechselfeldes, welches sich mittels einer Vorrichtung zur Detektion einer Magnetfeldänderung, insbesondere mittels der induktiven Detektions-Vorrichtung, als Kernspinresonanzsignal beobachten lässt. Unter Anregung von Atomkernen soll insbesondere verstanden werden, dass die Energie der eingestrahlten elektromagnetischen Felder, insbesondere Wechselfelder, eine Änderung der Kernspins der Atomkerne bewirkt. Ferner wird im Folgenden davon ausgegangen, dass insbesondere veränderliche Magnetfelder mit elektrischen Feldern gekoppelt sind (vgl. Maxwell-Gleichungen), sodass keine Unterscheidung zwischen elektrischem Feld und Magnetfeld vorgenommen wird. Zur Anregung von Kernspinresonanz-Effekten kommt es insbesondere auf die durch eine eingestrahlte elektromagnetische Strahlung übertragene Energie an. Vorteilhaft lässt sich diese Energie mittels gepulster elektromagnetischer Felder übertragen.

Unter "zur intrakorporalen Anordnung in einem menschlichen oder tierischen Körper vorgesehen und eingerichtet" ist zu verstehen, dass die Sensorvorrichtung eine biokompatible und biofunktionale Einhausung aufweist, sodass die Sensorvorrichtung von einem Körpergewebe, in dem sie implantiert ist, nicht derart abgestoßen oder eingenarbt wird, dass eine Funktionsbeeinträchtigung resultiert. Auf diese Weise kann ermöglicht werden, dass die Sensorvorrichtung in einen menschlichen oder tierischen Körper implantierbar ist, ohne dass eine Abwehrreaktion des Körpers auf die körperfremde Sensorvorrichtung erfolgt. Derartige biokompatible und biofunktionale Einhausungen sind dem Fachmann aus der Literatur bekannt. Entsprechend kann die Sensorvorrichtung im Körper implantiert sein und dort zur Durchführung einer NMR-Messung, d.h. zur Messung eines NMR-Spektrums bzw. von dafür relevanten Kernspinresonanzsignalen, verwendet werden. Dabei können erfindungsgemäß NMR-Spektroskopie-relevante Kernspinresonanzsignale betreffend das die Sensorvorrichtung unmittelbar umgebende oder durchdringende Medium erhalten werden. Unter "NMR-Spektroskopie-relevant" ist insbesondere zu verstehen, dass die ermittelten Kernspinresonanzsignale derart hochqualitativ ermittelbar sind, dass ein ermitteltes Zeitsignal fouriertransformiert und die enthaltenen Frequenzanteile nach Frequenz und Amplitude ausgewertet werden können. Eine derartige Auswertung im Rahmen der NMR-Spektroskopie setzt voraus, dass der Einfluss einer Inhomogenität des Magnetfelds B0 auf die Resonanzfrequenz besonders klein ist, um einen tatsächlichen Einfluss von Bindungszuständen und damit eine Molekülstruktur des die Sensorvorrichtung unmittelbar umgebenden oder durchdringenden Mediums messbar zu machen.

Aus den erhaltenen Kernspinresonanzsignalen der Sensorvorrichtung können Aussagen ableitbar sein, die unter anderem Entzündungsindikatoren, Krebsindikatoren, Nierenwerte, Mineralwerte, Drogen-Indikatoren, einen Blutzuckergehalt, einen Sauerstoffgehalt, einen Cholesteringehalt, Konzentrationen von Lipoprotein niedriger/hoher Dichte (LDL, HDL) oder andere Analyten oder dergleichen betreffen. Ferner können auch zeitlich-dynamische Prozesse chemischer Verbindungen, beispielsweise eine zeitliche Veränderung eines Blutzuckergehalts, ausgewertet werden. Insbesondere erlaubt das erfindungsgemäße NMR-Messgerät die Detektion, insbesondere die Detektion und Analyse, von Vitalkennwerten zumindest einer Umgebung der Sensorvorrichtung im menschlichen oder tierischen Körper ohne Probenentnahme. Insbesondere handelt es sich bei dem der Verwendung des NMR-Messgeräts zu Grunde liegenden Messverfahren der Kerspinspektroskopie um ein nicht zerstörendes Messverfahren, d.h. ein Vitalkennwert kann - nach der Implantation der Sensorvorrichtung - ohne weiteren invasiven/chrirugischen Eingriff in den Körper ermittelt werden.

Die Magnet-Vorrichtung der Sensorvorrichtung dient der Erzeugung des homogenen Magnetfelds B₀ in der zu untersuchenden Materialprobe, d.h. in dem zu untersuchenden Körperbereich. Die Magnet-Vorrichtung ist dazu eingerichtet, ein Magnetfeld B0 einer Magnetfeldstärke von mehr als 0.1 Tesla, insbesondere von mehr als 1.5 Tesla und ganz insbesondere von mehr als 5 Tesla bereitzustellen oder zu erzeugen. Das Magnetfeld B0 erstreckt sich in einem Volumen um die Magnet-Vorrichtung herum. Das durch die Magnet-Vorrichtung erzeugte Magnetfeld B0 dient der Ausrichtung der Kernspins der in dem Material des Körpers (in besagtem Volumen) vorhandenen Atomkerne in dem Sinne, dass sich die Kernspins auf Grund ihres magnetischen Kernspinmoments an den Magnetfeldlinien des Magnetfelds B0 ausrichten, insbesondere um die Magnetfeldlinien des Magnetfelds präzidieren. In einer Ausführungsform des NMR-Messgeräts weist die Magnet-Vorrichtung zumindest einen Elektromagnet und/oder zumindest einen Permanentmagnet auf, mittels dem/der das homogene Magnetfeld B0 erzeugbar ist. Unter Verwendung eines Permanentmagneten kann eine kostengünstige und dauerhaft funkionale Magnet-Vorrichtung realisiert werden, die - im Gegensatz zur Verwendung eines Elektromagneten - ohne eine weitere Energiequelle zu deren Betrieb auskommt. Insbesondere eignen sich zur Realisierung eines homogenen Magnetfelds B0 besonders starke Permanentmagnete, hergestellt aus Ferriten oder bevorzugt als Eisen-Cobalt-Nickel-Legierung oder besonders bevorzugt als Neodym-Eisen-Bor- oder Samarium-Cobalt-Legierung. Unter Verwendung eines Elektromagneten hingegen kann realisiert sein, dass durch elektrische Schaltung des Elektromagneten das Magnetfeld B₀ ein- und ausgeschaltet werden kann, sodass - im Gegensatz zur Verwendung eines Permanentmagneten - kein dauerhaftes Magnetfeld B0 im Körper vorhanden ist.

Eine Anregung der Kernspins erfolgt in Folge einer Einstrahlung von Energie in Form eines mittels der induktiven Detektions-Vorrichtung erzeugten Anregungssignals B1, d.h. in Form eines elektromagnetischen Feldes, insbesondere eines elektromagnetischen Wechselfeldes, beispielsweise eines gepulsten Magnetfeldes in zumindest einen Teilbereich desjenigen Bereichs des die Sensorvorrichtung umgebenden Materials, der ebenfalls von dem homogenen Magnetfeld B0 durchsetzt ist. Dabei definieren die elektromagnetischen Felder B0 und B1 in ihrem Überlagerungsfeld einen sensitiven Bereich der Sensorvorrichtung an derjenigen Stelle, an der die Felder senkrecht aufeinander stehen. In Abhängigkeit der Frequenz (Larmorfrequenz) des eingestrahlten Anregungssignals B1 (elektromagnetisches Feld) und der statischen Magnetfeldstärke des ersten Magnetfelds B1 wird der sensitive Bereich im Idealfall durch eine Fläche definiert, auf der die Magnetfeldstärke des ersten Magnetfelds konstant ist und insbesondere einen definierten Betrag aufweist. In einer Ausführungsform des NMR-Messgeräts umfasst die induktive Detektions-Vorrichtung zumindest eine RF-Spule zum Senden des Anregungssignals B1 und zumindest eine RF-Spule zum Empfangen des Kernspinresonanzsignals (d.h. des Echosignals). Auf diese Weise sind zumindest zwei RF-Spulen in der Sensorvorrichtung vorgesehen. Eine RF-Spule (Hochfrequenzspule) wird insbesondere mit einer Frequenz im Mega-Hertz-Bereich betrieben. Insbesondere liegt die Frequenz unter 900 Mega-Hertz, bevorzugt unter 200 Mega-Hertz und besonders bevorzugt unter 50 Mega-Hertz passt. Alternativ oder zusätzlich umfasst die induktive Detektions-Vorrichtung zumindest eine (einzelne) RF-Spule zum Senden des Anregungssignals B1 und zum Empfangen des Kernspinresonanzsignals. Auf diese Weise kann eine besonders kleinbauende Sensorvorrichtung realisiert werden, wobei auf Grund der besonders guten Überlagerung der elektromagnetischen Felder der sensitive Bereich besonders homogen realisierbar ist. In dieser Ausführungsform dient eine einzige RF-Spule der Erzegung des Anregungssignals B1 sowie der Detektion einer Magnetfeldänderung, wobei letztere es ermöglicht, mittels durch Kernspinrelaxation verursachte Magnetfeldänderungen auf materialspezifische Vitalkennwerte zu schließen. Ein Umklappen der Kernspins der Atomkerne, bei dem ein elektromagnetisches Feld emittiert wird, kann besonders vorteilhaft mittels derartiger RF-Spulen in Form einer durch die Magnetfeldvariation induzierten Spannung und/oder einen induzierten Strom detektiert werden. Diese Spannung und/oder dieser Strom kann von der elektrischen Schaltung des NMR-Messgeräts vorprozessiert, insbesondere ausgewertet, werden. In einer alternativen oder zusätzlichen Ausführungsform des NMR-Messgeräts kann die induktive Detektions-Vorrichtung statt der RF-Spule zum Empfangen des Kernspinresonanzsignals auch sogenannte SQUIDs (superconducting quantum interference device (dt. supraleitende Quanteninterferenzeinheiten)) umfassen, wobei Ausführungsformen ohne RF-Spule nicht in den Schutzumfang der Erfindung fallen.

Der sensitive Bereich kann insbesondere als ein schichtförmiger Bereich realisiert sein, der sich im Wesentlichen parallel und beabstandet zu einer Oberfläche der Sensorvorrichtung außerhalb der Sensorvorrichtung, insbesondere außerhalb eines Gehäuses der Sensorvorrichtung, erstreckt. Auf diese Weise kann erreicht werden, dass das Magnetfeld B0 und das Anregungssignal B1 aus der Sensorvorrichtung in das die Sensorvorrichtung umgebende Material eindringen und die NMR-Spektroskopiemessung in einer Umgebung der Sensorvorrichtung ermöglicht ist. In einer alternativen und/oder zusätzlichen Ausführungsform des Messgeräts kann die Sensorvorrichtung derart betrieben werden, dass sich der sensitive Bereich im Überlagerungsfeld der beiden Magnetfelder innerhalb der Sensorvorrichtung, insbesondere im Innern der induktiven Detektions-Vorrichtung, befindet. Weist die Sensorvorrichtung eine für das zu messende Medium (wie Blut) zumindest teilweise durchlässige Oberfläche auf, kann auf diese Weise konstruktiv einfach eine Sensorvorrichtung realisiert werden, die von dem zu messenden Medium durchsetzt oder durchspült wird.

In einer Ausführungsform kann die RF-Spule oder können die RF-Spulen ferner als Mikrospule(n) realisiert sein. Unter "Mikrospulen" sind besonders kleinbauende Spulen zu verstehen, deren Abmessungen geringer sind als 2 cm, insbesondere geringer als 1 cm, ganz insbesondere geringer als 0,5 cm. Derartige Mikrospulen lassen sich in nahezu beliebiger Windungszahl mit Hilfe von Druck oder Lithographie und galvanischer Abformung herstellen, wie dies aus dem Stand der Technik bekannt ist.

Unter einem "homogenen" Magnetfeld ist insbesondere zu verstehen, dass die das Magnetfeld beschreibenden Magnetfeldlinien sowie dessen lokale Magnetfeldstärke im sensitiven Bereich nur geringen, idealerweise keinen Variationen unterliegt und insbesondere keine Feldverzerrungen aufweist. Insbesondere kann ein lokal homogenes Magnetfeld unter Verwendung eines sehr kleinen sensitiven Bereichs - d.h. eines sehr kleinen Messvolumens, beispielsweise im Nanoliter-Bereich - erreicht werden. In einer alternativen oder zusätzlichen Ausführungsform des NMR-Messgeräts umfasst die Magnet-Vorrichtung eine Mehrzahl von Magneten, insbesondere ein Array von Elektromagneten und/oder ein Array von Permanentmagneten. Auf diese Weise kann ein besonders homogenes Magnetfeld B₀ erzeugt werden. Ferner kann ein derart homogenes Magnetfeld B₀ unter Verwendung zumindest einer Shim-Spule und/oder zumindest einer Schirmung erreicht werden.

In einer Ausführungsform des NMR-Messgeräts weist die induktive Detektions-Vorrichtung ein Array von RF-Spulen, ganz insbesondere ein Array von planaren RF-Spulen, auf. In einer Ausführungsform können derartige planare RF-Spulen auch gedruckt sein. Ein erfindungsgemäßes Array von RF-Spulen kann zur Erhöhung eines Signal-zu-Rausch-Verhältnisses verwendet werden. Ferner kann ein größerer homogener sensitiver Bereich der Sensorvorrichtung erzeugt werden.

In einer Ausführungsform des NMR-Messgeräts weisen die Magnet-Vorrichtung und die induktive Detektions-Vorrichtung eine vorgegebene, insbesondere feste, Anordnung zueinander auf. Insbesondere sind die Magnet-Vorrichtung und die induktive Detektions-Vorrichtung als ein zusammenhängendes Bauteil realisiert, beispielsweise auf einer Platine angeordnet, wobei die Anordnung unveränderlich ist. Es ist denkbar, dass ein Array von Magneten der Magnet-Vorrichtung und ein Array von RF-Spulen der induktiven Detektions-Vorrichtung derart übereinander platziert werden, dass eine Orthogonalität des Magnetfelds B0 und des Anregungssignals B1 in einem besonders großen Bereich gegeben ist.

Die Sensorvorrichtung weist eine anwendungsspezifische elektrische Schaltung zur Vorprozessierung von mittels der induktiven Detektions-Vorrichtung empfangenen Kernspinresonanzsignalen auf. Unter der elektrischen Schaltung kann dabei insbesondere ein ASIC verstanden werden. Die elektrische Schaltung dient der Steuerung zumindest der Sensorvorrichtung, wobei mittels der Schaltung ein Betrieb der Sensorvorrichtung ermöglicht wird. Insbesondere ist die elektrische Schaltung in einer Ausführungsform der Sensorvorrichtung mit zumindest einem Elektromagneten dazu vorgesehen, diesen Elektromagneten zu bestromen. Ferner ist die elektrische Schaltung dazu vorgesehen, die induktive Detektions-Vorrichtung zu steuern, insbesondere zu regeln und somit zu betreiben. Derart dient die elektrische Schaltung der Steuerung der induktiven Detektions-Vorrichtung, d.h. zur Steuerung der zumindest einen RF-Spule, wobei ermöglicht wird, Pulssequenzen des Anregungssignals B1 (d.h. des elektromagnetischen Felds) zu erzeugen, sodass das durch die induktive Detektions-Vorrichtung erzeugte Anregungssignal zeitlich und ortsabhängig modifiziert werden kann. Mittels der Pulssequenzen können besonders vorteilhaft die Kernspins der Atomkerne des Materials durch elektromagnetische Wechselfelder zur Absorption und Emission von Energiequanten, insbesondere zu Resonanz, angeregt werden. Ferner dient die elektrische Schaltung der Annahme und Verarbeitung von mittels der induktiven Detektions-Vorrichtung bereitgestellter Kernspinresonanzsignale. Die elektrische Schaltung weist zur Annahme der erfassten Kernspinresonanzsignale zumindest einen Informationseingang, eine Informationsverarbeitungseinheit zur Bearbeitung, insbesondere (Vor-)Auswertung und/oder (Vor-)Prozessierung der angenommenen Kernspinresonanzsignale, sowie eine Informationsausgabe zur Weitergabe der bearbeiteten und/oder ausgewerteten Kernspinresonanzsignale auf. Somit ist die elektrische Schaltung dazu eingerichtet, das Anregungssignal B1 (Sendesignal) und/oder ein Kernspinresonanzsignal (Empfangssignal) zu verarbeiten. Die elektrische Schaltung kann dafür beispielsweise einen rauscharmen Verstärker ("low noise amplifier", LNA) umfassen und/oder eine elektrische Schaltung, mittels der eine schnelle Fourier-Transformation ("fast Fourier transform", FFT) durchführbar ist. Die elektrische Schaltung gibt insbesondere ein prozessiertes verstärktes Kernspinresonanzsignal odere daraus bereits ausgewertete Größen, insbesondere ein NMR-Spektrum, eine Information betreffend einen Vitalkennwert, oder dergleichen aus. In einer Ausführungsform des NMR-Messgeräts weist die elektrische Schaltung eine Speichervorrichtung zur zumindest vorübergehenden Datenspeicherung auf, insbesondere zum Speichern von Messergebnissen und/oder empfangenen Kernspinresonanzsignalen. Diese Speichervorrichtung kann alle Formen elektrischer, insbesondere digitaler Speicher, umfassen, insbesondere Speicherchips wie USB-Sticks, Memory-Sticks, Speicherkarten, etc. Auf diese Weise kann ein NMR-Messgerät angegeben werden, das autark Kernspinresonanzsignale empfangen, verarbeiten und zumindest vorübergehend zwischenspeichern kann. Insbesondere wird ermöglich, kontinuierlich oder quasikontinuierlich Kernspinresonanzsignale aufzunehmen, wobei diese aber diskontinuierlich von der Sensorvorrichtung ausgelesen werden. Darüber hinaus ist denkbar, dass die elektrische Schaltung eine vollständige Steuervorrichtung der Sensorvorrichtung darstellt, die Mittel zur Kommunikation mit anderen Komponenten des NMR-Messgeräts, beispielsweise mit einer extrakorporalen Datenverarbeitung in Form einer Auswertevorrichtung, mit einer Datenkommunikationsvorrichtung, mit einer Ausgabevorrichtung, mit einer Eingabevorrichtung oder dergleichen, aufweist. Ferner ist denkbar, dass die elektrische Schaltung Komponenten wie einen Prozessor, einen Speicher und ein Betriebsprogramm mit Auswerte- und Berechnungsroutinen umfasst. Derart kann die elektrische Schaltung dazu vorgesehen sein, die von der induktiven Detektions-Vorrichtung erhaltenen Kernspinresonanzsignale auszuwerten und daraus zumindest Informationen betreffend einen Vitalkennwert abzuleiten. In einer Ausführungsform des NMR-Messgeräts können die Bauteile (d.h. Magnet-Vorrichtung, induktive Detektions-Vorrichtung, elektrische Schaltung, ggf. Speichervorrichtung etc.) auf einer Platine (Leiterplatte) angeordnet sein, bevorzugt zumindest teilweise in Form eines Mikrokontrollers.

In einer Ausführungsform des NMR-Messgeräts weist dieses eine Ausgabevorrichtung auf, die der intrakorporalen Sensorvorrichtung zugehörig realisiert ist. Unter einer "Ausgabevorrichtung" soll zumindest ein Mittel verstanden werden, das dazu vorgesehen ist, zumindest eine wechselnde Information akustisch, optisch und/oder taktil auszugeben. Dies kann beispielsweise mittels eines Displays, eines Touch-Displays, eines Tonsignals, einer Veränderung eines Betriebsparameters, eines Vibrationsgebers und/oder einer LED-Anzeige realisiert werden. Beispielsweise ist denkbar, in der Sensorvorrichtung einen Vibrationsgeber vorzusehen. Derart kann insbesondere ein NMR-Messgerät realisiert werden, das ohne weitere extrakorporale Ausgabevorrichtung auskommt.

In einer alternativen oder zusätzlichen Ausführungsform des NMR-Messgeräts weist dieses eine extrakorporal dem NMR-Messgerät zugehörige und mit der Sensorvorrichtung über eine Datenkommunikationsschnittstelle verbindbare Ausgabevorrichtung auf. Insbesondere können auszugebende Informationen, beispielsweise Auswerteergebnisse und/oder Informationen betreffend einen Betriebszustand des NMR-Messgeräts, an eine extrakorporale Steuervorrichtung und/oder an ein Daten verarbeitendes System ausgegeben werden. Letzteres umfasst zumindest eine Ausgabe einer Information an einen extrakorporalen Teil des NMR-Messgeräts und/oder an ein externes Geräte wie ein Smartphone, ein Tablet-PC, ein PC sowie an ein anderes, einem Fachmann als sinnvoll erscheinendes externes Datengerät, das über eine Datenkommunikationsschnittstelle mit der Sensorvorrichtung, insbesondere mit der elektrischen Schaltung der Sensorvorrichtung, verbindbar ist. Die Datenkommunikationsschnittstelle kann ein standardisiertes Kommunikationsprotokoll zu einer Übertragung von elektronischen, insbesondere digitalen Daten, nutzen. Die Datenkommunikationsschnittstelle umfasst eine drahtlose Schnittstelle, insbesondere beispielsweise eine WLAN-, Bluetooth-, Infrarot-, NFC-, RFID-Schnittstelle oder eine andere, einem Fachmann als sinnvoll erscheinende drahtlose Schnittstelle.

Erfindungsgemäß ist die induktive Detektions-Vorrichtung induktiv mit einem extrakorporalen Signalgenerator des NMR-Messgeräts zur Einkopplung eines Anregungssignals und optional zur Auskopplung eines Kernspinresonanzsignals koppelbar. Auf diese Weise kann die Sensorvorrichtung besonders kleinbauend realisiert sein, da eine aufwändige Schaltung zur Erzeugung des Anregungssignals im extrakorporalen Teil des NMR-Messgeräts angeordnet werden kann. Das NMR-Messgerät weist zur induktiven Kopplung geeignete Mittel auf, mittels der das von dem extrakorporalen Signalgenerator erzeugte Anregungssignal drahtlos in einen von der induktiven Detektions-Vorrichtung mittels zumindest einer RF-Spule gebildeten Schwingkreis, einkoppelbar ist und optional das empfangene Kernspinresonanzsignal von der induktiven Detektions-Vorrichtung an den extrakorporalen Teil des NMR-Messgeräts auskoppelbar ist.

In einer Ausführungsform des NMR-Messgeräts ist die Sensorvorrichtung induktiv mit einer extrakorporalen Energieversorgungsvorrichtung koppelbar, sodass in der Sensorvorrichtung eine Stromversorgung zum Betrieb der Sensorvorrichtung induzierbar ist. Eine Energieversorgungsvorrichtung ist zumindest dazu vorgesehen, die Sensorvorrichtung, insbesondere das gesamte NMR-Messgerät, zur Inbetriebnahme und während des Betriebs mit elektrischer Energie zu versorgen. Auf diese Weise kann die Sensorvorrichtung besonders kleinbauend realisiert sein, da auf eine intrakorporale Energieversorgungsvorrichtung der Sensoreinheit verzichtet und diese in den extrakorporalen Teil des NMR-Messgeräts ausgelagert werden kann. Das NMR-Messgerät weist zur induktiven Kopplung geeignete Mittel auf, mittels der die von einer extrakorporalen Energieversorgungsvorrichtung, beispielsweise einem Akkumulator, bereitgestellte Energie drahtlos an die Sensorvorrichtung übertragbar ist. In einem Ausführungsbeispiel handelt es sich bei der Energieversorgungsvorrichtung um einen stromnetzunabhängigen Energiespeicher, insbesondere einen Akkumulator, eine Batterie, eine Brennstoffzelle, einen Kondensator, einen anderweitigen, dem Fachmann sinnvoll erscheinenden Energiespeicher oder eine Kombination/Mehrung derer. Insbesondere eigenen sich zur Energieversorgung insbesondere Akkumulatoren mit einer Zellchemie, die eine hohe Leistungs- und/oder Energiedichte bereitstellt. Dazu gehören derzeit beispielsweise Akkumulatoren der Lithium- und Lithium-lonen-Zellchemie, insbesondere Lithium- Eisenphosphat-, Lithium-Manganoxid-, Lithium-Nickel-Cobalt-Mangan-Oxid-, überlithiierte Lithium-Nickel-Cobalt-Mangan-Oxid-, Lithium-Schwefel-, Lithium-Polymer- und Lithium-Sauerstoff-Akkumulatoren. Es sei angemerkt, dass die von der extrakorporalen Energieversorgungsvorrichtung übertragene Energie in der Sensorvorrichtung sofort zum Betrieb der Sensorvorrichtung genutzt werden kann oder auch in der Sensorvorrichtung zwischengespeichert werden kann, beispielsweise in einem kleinen Akkumulator oder einem Kondensator.

In einer alternativen oder zusätzlichen Ausführungsform des NMR-Messgeräts ist die Sensorvorrichtung elektrisch mit einer intrakorporalen Energieversorgungsvorrichtung gekoppelt, die eine Stromversorgung zum Betrieb der Sensorvorrichtung bereitstellt. Auf diese Weise kann eine autark betreibbare Sensorvorrichtung bereitgestellt werden, die insbesondere ohne eine extrakorporal vorgesehene Energieversorgungsvorrichtung zumindest vorübergehend funktionsfähig sein kann. Vorteilhaft kann so beispielsweise eine über einen längeren Zeitraum durchgeführte Messung ("Dauermessung") ermöglicht werden, wobei ein Anwender des NMR-Messgeräts, insbesondere der Sensorvorrichtung, sich dabei unabhängig von einer extrakorporalen Energieversorgungsvorrichtung bewegen kann. Unter einer intrakorporalen Energieversorgungsvorrichtung kann beispielsweise eine Batterie oder ein Akkumulator, ebenso aber auch eine auf anderweitigigen, insbesondere elektrochemischen, thermoelektrischen, elektromagnetischen oder biomechanischen Konzepten beruhende Energieversorgungsvorrichtung verstanden werden. In einem Ausführungsbeispiel kann eine derartige intrakorporale Energieversorgungsvorrichtung beispielsweise unter Verwendung eines leistungsstarken Hybrid-Superkondensators realisiert sein, der mittels einer Glukose-Brennstoffzelle in Zeiten, in denen keine Messung stattfindet, wieder geladen wird.

Ferner kann das erfindungsgemäße NMR-Messgerät eine Eingabevorrichtung zur Eingabe von Arbeitsparametern aufweisen. Unter einer Eingabevorrichtung soll dabei insbesondere ein Mittel verstanden werden, das dazu vorgesehen ist, zumindest eine Information von einem Bediener des Messgeräts (dann insbesondere Benutzerschnittstelle) und/oder einem anderen Gerät über eine akustische, optische, gestengestützte und/oder taktile Eingabe anzunehmen und an die Steuervorrichtung des Messgeräts weiterzuleiten. In einem Ausführungsbeispiel ist die Eingabevorrichtung der Sensorvorrichtung zugehörig in oder an der Sensorvorrichtung angeordnet, beispielsweise als drucksensitiver Tastschalter, mittels dem die Durchführung einer Messung ausgelöst werden kann. In einem alternativen Ausführungsbeispiel kann die Eingabevorrichtung auch extrakorporal dem extrakorporalen Teil des NMR-Messgeräts zugehörig realisiert sein.

Ferner wird ein Verfahren zur Verwendung eines erfindungsgemäßen NMR-Messgeräts zur intrakorporalen NMR-spektroskopischen Untersuchung eines menschlichen oder tierischen Körpers gemäß Anspruch 10 vorgeschlagen.

### Zeichnungen

Die Erfindung ist anhand von in den Zeichnungen dargestellten Ausführungsbeispielen in der nachfolgenden Beschreibung näher erläutert. Die Zeichnung, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen, wobei der Schutzumfang der Erfindung durch die Ansprüche definiert ist.

Gleiche oder ähnliche Bezugszeichen in den Figuren bezeichnen gleiche oder ähnliche Elemente.

Es zeigen:
Figur 1 schematische Schnittansicht einer Ausgestaltung eines NMR-Messgeräts welches nicht in den Schutzumfang der vorliegenden Erfindung fällt;
Figur 2 schematische Schnittansicht einer alternativen Ausgestaltung eines NMR-Messgeräts welches nicht in den Schutzumfang der vorliegenden Erfindung fällt;
Figur 3 schematische Schnittansicht einer alternativen Ausgestaltung eines NMR-Messgeräts welches nicht in den Schutzumfang der vorliegenden Erfindung fällt.

### Beschreibung der Ausführungsbeispiele

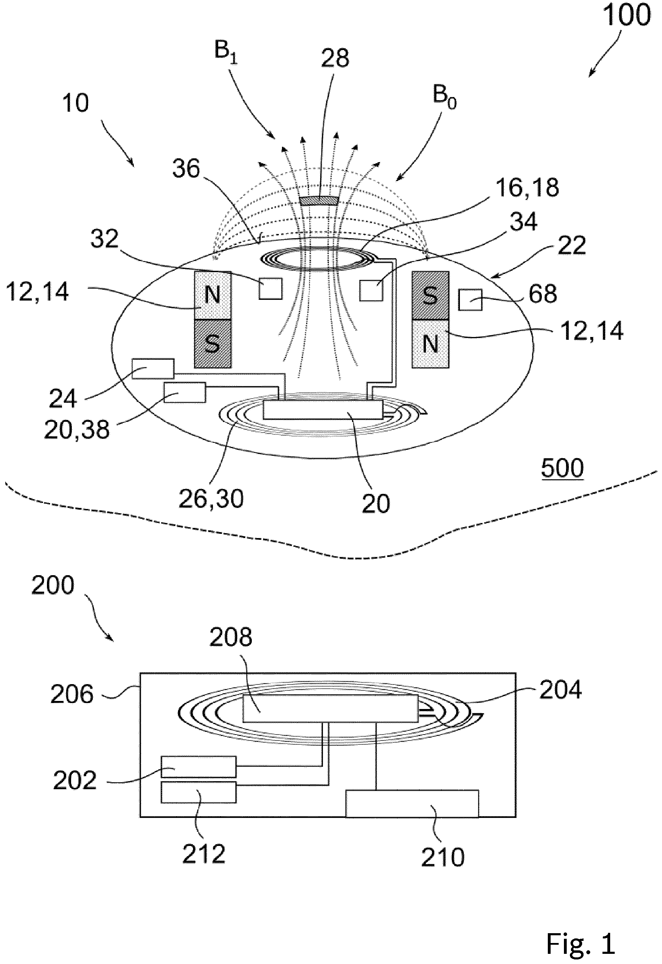

Figur 1, Figur 2 und Figur 3 zeigen jeweils Ansichten einer beispielhaften Ausführungsform eines NMR-Messgeräts 100 außerhalb des Schutzumfangs der Erfindung in schematischer seitlicher Darstellung. Das jeweils dargestellte NMR-Messgerät 100 umfasst dabei jeweils eine in einen Körper 500 implantierbare Sensorvorrichtung 10 und einen extrakorporal angeordneten Teil 200 des NMR-Messgeräts 100. Die zur intrakorporalen Anordnung in einem menschlichen oder tierischen Körper 500 vorgesehene Sensorvorrichtung 10 weist dabei ein biokompatibles und biofunktionales Gehäuse 22 auf.

Die Sensorvorrichtung 10 weist eine Magnet-Vorrichtung 12 zur Erzeugung eines homogenen Magnetfelds B0 auf, die in der Ausführungsform der Figur 1 zwei Permanentmagnete 14 umfasst. Die zwei Permanentmagnete 14 sind antiparallel zueinander und jeweils im Wesentlichen senkrecht zu einem Teil der Oberfläche 36 des Gehäuses 22 ausgerichtet, sodass sie ein Magnetfeld B0 erzeugen, das im Wesentlichen parallel zu besagtem Teil der Oberfläche 36 des Gehäuses 22 ausgerichtet ist. Dieses zur Ausrichtung der Kernspins der in der Materialprobe (d.h. im Körper 500) vorhandenen Atomkerne vorgesehene Magnetfeld B0 weist beispielhaft eine Magnetfeldstärke von 0.5 Tesla auf, wobei die Permanentmagnete 14 aus einer Neodym-Eisen-Bor-Legierung hergestellt sind.

Eine induktive Detektions-Vorrichtung 16 ist zum Senden eines Anregungssignals B1 und zum Empfangen eines Kernspinresonanzsignals vorgesehen. Die induktive Detektions-Vorrichtung 16 des dargestellten Ausführungsbeispiels umfasst eine RF-Spule 18 (in einem alternativen, hier nicht dargestellten Ausführungsbeispiel könnte die induktive Detektions-Vorrichtung 16 auch eine RF-Spule 18 zum Senden des Anregungssignals B1 und eine weitere RF-Spule 18 zum Empfangen des Kernspinresonanzsignals aufweisen). Sobald durch diese RF-Spule 18 Strom fließt, wird ein elektromagnetisches Feld - das Anregungssignal B1 - induziert. Die RF-Spule 18 ist als planare, insbesondere gedruckte Spule realisiert, die sich in einer Ebene im Wesentlichen parallel zum besagten Teil der Oberfläche 36 des Gehäuses 22 erstreckt.

Die Magnet-Vorrichtung 12, insbesondere die Permanentmagnete 14, und die induktive Detektions-Vorrichtung 16, insbesondere die RF-Spule 18, weisen zueinander eine feste Anordnung auf. Die feste Anordnung bewirkt, dass die durch die Magnet-Vorrichtung 12 und die induktive Detektions-Vorrichtung 16 erzeugten elektromagnetischen Felder, d.h. das Magnetfeld B0 und das Anregungssignal B1, im Wesentlichen senkrecht aufeinander stehen. Das Anregungssignal B1 kann dabei ebenfalls als Magnetfeld interpretiert werden, dessen Magnetfeldlinien (Pfeile) im Wesentlichen senkrecht zu den Magnetfeldlinien (gestrichelte Linien) des durch die Permanentmagnete 14 erzeugten Magnetfeldes B0 ausgerichtet ist. Die beiden Magnetfelder B0 und B1 überlagern sich daher in einem ausgedehnten Bereich, der im Wesentlichen außerhalb des Gehäuses 22 der Sensorvorrichtung 10 liegt. In diesem Bereich befindet sich insbesondere auch der sensitive Bereich 28 als insbesondere schichtförmiger Bereich. Die Krümmung und Ausformung des Magnetfelds B0 und damit des sensitiven Bereichs 28 kann unter Verwendung weiterer Mittel, beispielsweise einer Shim-Spule 32 und einer magnetischen Schirmung 34, beeinflusst und insbesondere homogenisiert werden.

Der derart realisierte und implantierbare Sensor zur Durchführung einer NMR-Spektroskopie ist zur Anregung einer Kernspinresonanz in Atomkernen des die Sensorvorrichtung 10 umgebenden Materials, d.h. des Körpers 500, vorgesehen. Zur intrakorporalen Messung von NMR-Spektren mittels der Sensorvorrichtung 10 wird die Sensorvorrichtung 10 daher in einen menschlichen oder tierischen Körper 500 implantiert. Dabei dringen die durch die Magnet-Vorrichtung 12 und die induktive Detektions-Vorrichtung 16 erzeugten elektromagnetischen Felder, insbesondere das homogene Magnetfeld B0 und das Anregungssignal B1, durch die Oberfläche 36 des Gehäuses 22 aus der Sensorvorrichtung 10 aus und in das Material des Körpers 500 ein, wobei der sensitiver Bereich 28 in dem Körper 500 zu liegen kommt. Magnetfeldänderungen in Folge eines Kernspinresonanzeffekts der in dem Material des Körpers 500 angeregten Kernspins der Atomkerne, d.h. verursacht durch Absorption und/oder Emission elektromagnetischer Felder durch die Atomkerne einhergehend mit einer Änderung deren Energiezustände, kann mittels der induktiven Detektions-Vorrichtung 16, insbesondere mittels der RF-Spule 18, detektiert werden. Dieses Kernspinresonanzsignal (Messsignal), insbesondere dessen Amplitude und Relaxationszeiten, wird an eine anwendungsspezifische elektrische Schaltung 20 weitergeleitet, von der es zumindest vorprozessiert und/oder aufbereitet, bevorzugt ausgewertet, wird.

Die anwendungsspezifische elektrische Schaltung 20 ist dabei zumindest zur Vorprozessierung von mittels der induktiven Detektions-Vorrichtung 16 empfangenen Kernspinresonanzsignalen eingerichtet. Die elektrische Schaltung 20 weist eine Steuerelektronik umfassend Mittel zur Kommunikation mit den Komponenten der Sensorvorrichtung 10 auf, insbesondere Mittel zur Steuerung und Regelung der induktiven Detektions-Vorrichtung 16 sowie einer Ausgabevorrichtung 24. Die elektrische Schaltung 20 umfasst ferner zur zumindest vorübergehenden Zwischenspeicherung der zumindest vorprozessierten Kernspinresonanzsignale eine Speichervorrichtung 38. Die Speichervorrichtung 38 ist hier beispielhaft als ein Flash-Speichermedium realisiert. Die elektrische Schaltung 20 ist zur Auswertung von von der induktiven Detektions-Vorrichtung 16 gelieferten Kernspinresonanzsignalen (Messsignalen) vorgesehen und weist dafür einen Informationseingang, eine Informationsverarbeitung und einen Informationsausgang auf. Die elektrische Schaltung 20 führt auf Grundlage der erhaltenen Kernspinresonanzsignale eine Vorverstärkung und eine schnelle Fouriertransformation (FFT) durch.

In einem alternativen, hier nicht näher dargestellten Ausführungsbeispiel weist die elektrische Schaltung 20 Mittel auf, mittels der die induktive Detektions-Vorrichtung 16 induktiv mit einem extrakorporalen Signalgenerator des NMR-Messgeräts 100 (beispielsweise integriert in die Steuer- und Auswertevorrichtung 208 des NMR-Messgeräts 100) zur Einkopplung eines Anregungssignals koppelbar ist. Eine derartige Kopplung kann beispielsweise unter Verwendung einer Antennenanordnung realisiert sein.

In einem alternativen, hier nicht näher dargestellten Ausführungsbeispiel weist die elektrische Schaltung 20 zumindest einen Prozessor mit einem Speicher und mit darauf gespeicherten und ausführbaren Betriebs- und/oder Auswerteprogrammen auf. Die Betriebs- und/oder Auswerteprogamme erlauben, ein empfangenes Kernspinresonanzsignal auszuwerten und Informationen betreffend die Detektion und/oder Analyse und/oder Unterscheidung von Materialkomponenten des Körpers 500 zu bestimmen. Dazu können die Betriebs- und/oder Auswerteprogramme ferner beispielsweise gespeicherte Korrektur- und/oder Kalibriertabellen aufweisen, die es erlauben, die Auswerteergebnisse zu interpretieren, umzurechnen und/oder zu inter- und/oder extrapolieren.

Ein Messergebnis wird dem Nutzer mittels der Ausgabevorrichtung 24 der Sensorvorrichtung 10 ausgegeben. Die Ausgabevorrichtung 24 zur Ausgabe von Informationen, insbesondere zur Ausgabe von Warnungen hinsichtlich einer Glukosekonzentration, ist in Form eines Vibrationsgebers realisiert. Darüber hinaus wird ein Messergebnis über eine Datenkommunikationsschnittstelle 30, hier realisiert durch eine Spulen-Anordnung 26 (d.h. als induktive Antenne), an einen extrakorporalen Teil 200 des NMR-Messgeräts 100 - hier realisiert als ein handgehaltenes Auslesegerät -und/oder an ein weiteres externes Datenverarbeitungsgerät gesendet.

Zur Energieversorgung der Sensorvorrichtung 10 dient die im Inneren der Sensorvorrichtung 10 integrierte Spulen-Anordnung 26. Mittels der Spulen-Anordnung 26 kann die Sensorvorrichtung 10 induktiv mit einer in dem extrakorporalen Teil 200 des NMR-Messgeräts 100 angeordneten Energieversorgungsvorrichtung 202 gekoppelt werden, sodass in der Sensorvorrichtung 10 eine Stromversorgung zum Betrieb der Sensorvorrichtung 10 induzierbar ist - entweder in Form eines induktiven Ladens, d.h. zur Aufladung einer weiteren Energiespeichervorrichtung der Sensorvorrichtung 10 (hier nicht näher dargestellt), oder während des Betriebs, d.h. ohne zusäztliche Energiespeichervorrichtung. Zur induktiven Kopplung weist der extrakorporale Teil 200 des NMR-Messgeräts 100 ebenfalls eine Spulen-Anordnung 204 zur auf. In einem alternativen Ausführungsbeispiel (hier nicht näher dargestellt) kann die Sensorvorrichtung 10 auch elektrisch mit einer intrakorporalen Energieversorgungsvorrichtung gekoppelt sein, die eine Stromversorgung zum Betrieb der Sensorvorrichtung 10 bereitstellt.

Die Komponenten der Magnet-Vorrichtung 12, der induktiven Detektions-Vorrichtung 16, der elektrischen Schaltung 20 und der Spulen-Anordnung 26 sind auf einem Trägerelement, insbesondere einer Systemplatine oder Leiterplatte, innerhalb des Gehäuses 22 der Sensorvorrichtung angeordnet und miteinander verschaltet (hier nicht näher dargestellt).

Der extrakorporale Teil 200 des NMR-Messgeräts 100 weist ein Gehäuse 206 auf, in dem eine Steuer- und Auswertevorrichtung 208, eine Ein- und Ausgabevorrichtung 210 in Form eines berührungssensitiven Bildschirms, eine Energieversorgungsvorrichtung 202, eine Datenspeichervorrichtung 212 sowie die Spulen-Anordnung 204 untergebracht sind. Wie bereits beschrieben dient die Spulen-Anordnung 204 der Übertragung einer Betriebsenergie aus der Energieversorgungsvorrichtung 202 an die Sensorvorrichtung 10, insbesondere an die elektrische Schaltung 20. Ferner dient die Spulen-Anordnung 204 der drahtlosen Übertragung von Kernspinresonanzsignalen von der Sensorvorrichtung 10 unter Verwendung der zur Spulen-Anordnung 204 kompatiblen Spulen-Anordnung 26 der Sensorvorrichtung 10. Die Steuer- und Auswertevorrichtung 208 dient der Annahme und Weiterverarbeitung der von der Sensorvorrichtung 10 übertragenen (vorprozessierten) Kernspinresonanzsignale. Ausgewertete Informationen können einem Benutzer des NMR-Messgeräts 100 unter Verwendung der Ein- und Ausgabevorrichtung 210 ausgegeben werden. Die Ausgabe auf dem Bildschirm kann dabei grafisch, numerisch und/oder alphanumerisch, beispielsweise in Form eines Messwerts, einer Messkurve, eines Signalverlaufs, eines Zeitverlauf, als Bilddaten oder in einer Gradientendarstellung sowie in einer Kombination derer erfolgen. Alternativ oder zusätzlich ist eine Darstellung mittels einer Signalanzeige möglich, insbesondere beispielsweise einer Leuchtdiode, die beispielsweise über eine Farbcodierung (z.B. rot, gelb, grün) eine Zielgröße bewertet. Somit weist das NMR-Messgerät 100 eine Ausgabevorrichtung 24, die der intrakorporalen Sensorvorrichtung 10 zugehörig realisiert ist, und eine dem extrakorporalen Teil 500 des NMR-Messgeräts 100 zugehörige Ein- und Ausgabevorrichtung 210 auf, wobei letztere mit der Sensorvorrichtung 10 über eine Datenkommunikationsschnittstelle, hier realisiert als Spulen-Anordnungen 26, 204, verbindbar realisiert ist.

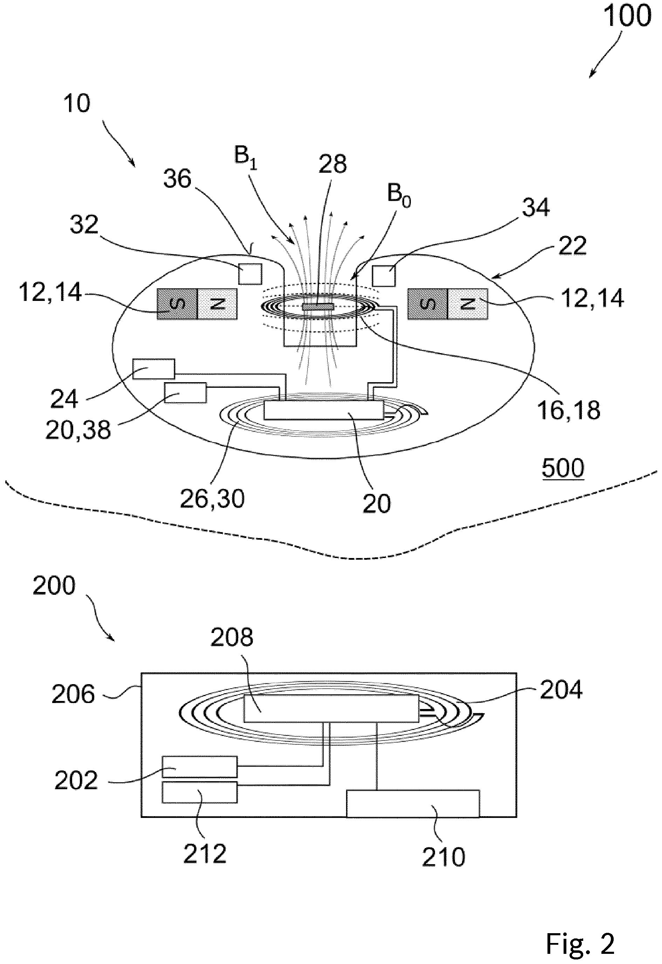

In Figur 2 ist ein alternatives Ausführungsbeispiel eines NMR-Messgeräts 100 in schematischer seitlicher Darstellung wiedergegeben. Das dargestellte NMR-Messgerät 100 umfasst dabei ebenfalls eine in einen Körper 500 implantierbare Sensorvorrichtung 10 und einen extrakorporal angeordneten Teil 200. Im Unterschied zum Ausführungsbeispiel der Figur 1 ist die Anordnung der Magnet-Vorrichtung 12 und der induktiven Detektions-Vorrichtung 16 verändert. Insbesondere weist die Magnet-Vorrichtung 12 zwei kollinear angeordnete Permanentmagnete 14 (in Nord-Süd/Nord-Süd-Abfolge) auf. Die Magnet-Vorrichtung 12 erzeugt dabei ein statisches Magnetfeld B0, das im Wesentlichen im Innenbereich des durch das Gehäuse 22 geformten Rotationsellipsoids liegt. Das Gehäuse 22 weist dabei eine einseitige, zylindrische Vertiefung auf. Die induktive Detektions-Vorrichtung 16 umfasst ebenfalls wie in Figur 1 eine RF-Spule 18, die zwischen den beiden Permanentmagneten 14 angeordnet ist und deren Wicklungsebene kollinear zur Erstreckungsrichtung der Permanentmagnete 14 liegt. Das durch die RF-Spule 18 im Falle einer Bestromung der RF-Spule 18 erzeugbare Anregungssignal B1 ist im Wesentlichen senkrecht zu dem Magnetfeld B0 ausgerichtet. Die beiden Magnetfelder überlagern sich in einem Bereich, der im Wesentlichen innerhalb der von dem Gehäuse 22 gebildeten Vertiefung liegt und der daher von mehreren Seiten durch das Gehäuse 22 umschlossen ist und an einer Seite zum Medium hin offen ist. Die auf diese Weise vorgeschlagene Anordnung der Magnet-Vorrichtung 12 und der induktiven Detektions-Vorrichtung 16 erlaubt es, ein noch homogeneres Magnetfeld B0 zu realisieren. In einem weiteren Ausführungsbeispiel (hier nicht näher dargestellt) ist denkbar, dass ein Pumpsystem das derart realisierte Messvolumen, d.h. die durch das Gehäuse 22 gebildete Vertiefung, mit Blut oder Interstitialflüssigkeit oder dergleichen durchspült.

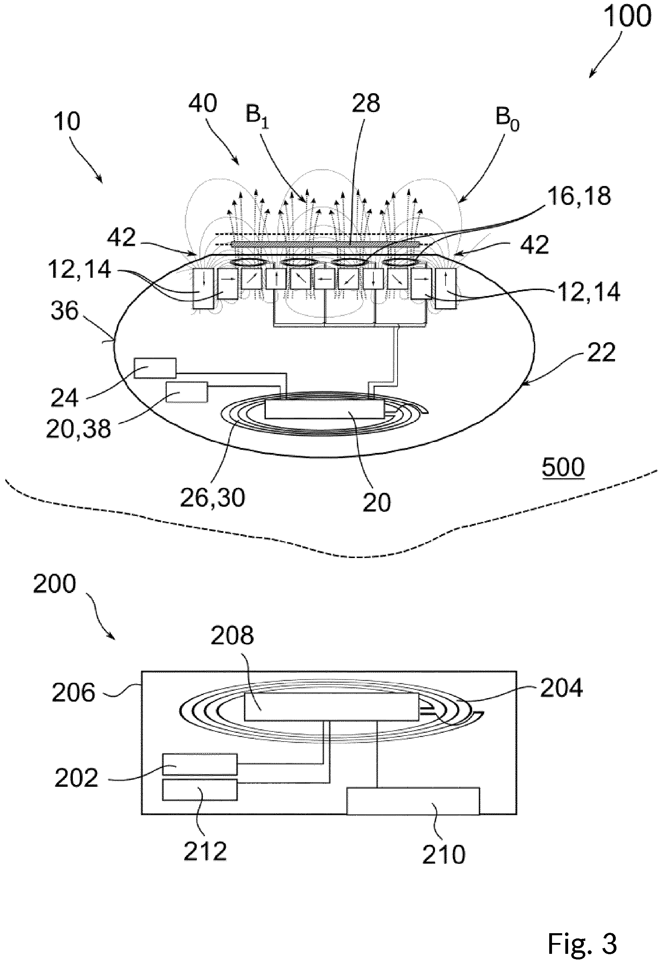

In Figur 3 ist ein alternatives Ausführungsbeispiel eines NMR-Messgeräts 100 in schematischer seitlicher Darstellung wiedergegeben. Das dargestellte NMR-Messgerät 100 umfasst dabei ebenfalls eine in einen Körper 500 implantierbare Sensorvorrichtung 10 und einen extrakorporal angeordneten Teil 200. Im Unterschied zum Ausführungsbeispiel der Figur 1 ist die Anordnung der Magnet-Vorrichtung 12 und der induktiven Detektions-Vorrichtung 16 verändert. Insbesondere weist die Magnet-Vorrichtung 12 ein Array von Permanentmagneten 14 auf (gekennzeichnet durch die kleinen Pfeile), das aus einer planaren Anordnung mehrerer in einer Ebene nebeneinander angeordneter Permanentmagnete 14 zusammengesetzt ist (in einem alternativen Ausführungsbeispiel kann es sich hierbei auch um ein Array von Elektromagneten handeln). Die Permanentmagnete 14 sind quaderförmig ausgebildet und weisen quadratische Querschnitte auf. Grundsätzlich können die Permanentmagnete 14 jedoch auch andere geeignete Formen aufweisen, zum Beispiel zylindrische oder runde. Die spezielle Anordnung der Permanentmagnete 14 innerhalb der Magnet-Vorrichtung 12 erzeugt ein Magnetfeld B0 mit definierten und besonders homogenen Eigenschaften innerhalb eines bestimmten Raumbereichs 40 (hier in der Figur 3 oberhalb der Sensorvorrichtung 10 außerhalb der Oberfläche 36). Der Raumbereich 40 definiert dabei ein Nutzvolumen, welches durch eine besonders hohe Magnetfeldhomogenität des Magnetfelds B0 charakterisiert ist. Zur Verdeutlichung zeigt Figur 3 die Orientierungen der Magnetisierung der Permanentmagnete 14 mittels entsprechender Pfeile (jeweils in den Permanentmagneten 14). Hierbei wird deutlich, dass die Magnetisierungsrichtungen von unmittelbar nebeneinander benachbarten Permanentmagneten 14 jeweils um einen bestimmten Winkel zueinander verdreht sind. Durch die Drehung der Orientierung der Magnetisierung benachbarter Permanentmagnete 14 wird eine spezielle Magnetanordnung erreicht, welche ermöglicht, dass sich der magnetische Fluss auf einer ersten Seite der Magnet-Vorrichtung 12 fast aufhebt (hier im Innern der Sensorvorrichtung 10), während er auf der der ersten Seite gegenüber liegenden zweiten Seite (hier im Außenbereich oberhalb der Sensorvorrichtung 10 in Figur 3, d.h. im Raumbereich 40) verstärkt wird. Somit wird das Magnetfeld B0, welches in der Figur 3 schematisch mittels der Feldlinien angedeutet ist, hauptsächlich auf der dem Raumbereich 40 zugewandten Seite der planaren Magnet-Vorrichtung 12 konzentriert. Im vorliegenden Ausführungsbeispiel beträgt der Drehwinkel für die Permanentmagnete 14 jeweils 45° (außer bei den beiden äußeren Permanentmagneten 14, dort 90°). Insbesondere kann der Drehwinkel zwischen benachbarten Permanentmagneten 14 in Abhängigkeit der jeweiligen Anwendung variieren. Insbesondere kann bei Verwendung einer größeren Anzahl separater Permanentmagnete 14 ein kleinerer Drehwinkel verwendet werden als bei einer kleineren Anzahl von Permanentmagneten 14. Um eine Abschwächung des statischen Magnetfelds B0 in den Randbereichen 42 der planaren Magnet-Vorrichtung 12 zu kompensieren, können in den entsprechenden Randbereichen 42 stärkere Permanentmagnete 14 verwendet werden als in einem mittleren Bereich der Magnet-Vorrichtung 12, hier dargestellt durch größer bzw. höher ausgebildete Permanentmagnete 14. Auf diese Weise wird eine vorteilhafte Homogenität des Magnetfelder B0 erreicht, die es ermöglicht, NMR-Spektroskopie intrakorporal mit einer derart kleinbauenden Sensorvorrichtung 10 durchzuführen.

Ferner ist ebenfalls die induktive Detektions-Vorrichtung 16 als ein Array von RF-Spulen 18 realisiert, insbesondere als ein Array von planaren RF-Spulen, wobei die RF-Spulen 18 zwischen der Oberfläche 36 des Gehäuses 22 der Sensorvorrichtung 10 und der Magnet-Vorrichtung 12 angeordnet sind. Durch die vorgeschlagene Anordnung der RF-Spulen 18 kann erreicht werden, dass eine Orthogonalität des Magnetfelds B0 und des Anregungssignals B1 in einem besonders großen Bereich gegeben ist, sodass sich der sensitive Bereich 28 über einen besonders großen Bereich erstreckt.

## Patentansprüche

1. NMR-Messgerät (100), umfassend zumindest eine zu einer intrakorporalen Anordnung in einem menschlichen oder tierischen Körper (500) speziell eingerichtete Sensorvorrichtung (10) und umfassend zumindest einen nicht biokompatiblen und nicht biofunktionalen anderen Teil (200) welcher zur extrakorporalen Anordnung eingerichtet ist,
wobei die Sensorvorrichtung (10) zumindest aufweist:
• eine Magnet-Vorrichtung (12) zur Erzeugung eines homogenen Magnetfelds B0,
• eine induktive Detektions-Vorrichtung (16) zum Senden eines Kernspinresonanz-Anregungssignals und
zum Empfangen eines Kernspinresonanzsignals, wozu die induktive Detektions-Vorrichtung (16) zumindest eine RF-Spule (18) aufweist, und
• eine anwendungsspezifische elektrische Schaltung (20) zumindest zur Vorprozessierung von mittels der induktiven Detektions- Vorrichtung (16) empfangenen Kernspinresonanzsignalen, und
• ein biokompatibles und biofunktionales Gehäuse (22),
wobei der andere Teil (200) einen Signalgenerator mit einer Schaltung zu einer Erzeugung des Anregungssignals aufweist,
wobei das NMR-Messgerät (100) zur intrakorporalen Messung von NMR-Spektren mittels der Sensorvorrichtung (10) eingerichtet ist,
wobei die induktive Detektions-Vorrichtung (16) der Sensorvorrichtung (10) induktiv mit dem Signalgenerator des NMR-Messgeräts (100) zur Einkopplung des Anregungssignals koppelbar ist, wenn dieser extrakorporal angeordnet ist, und wobei das NMR-Messgerät (100) zur induktiven Kopplung geeignete Mittel aufweist, mit denen das von dem extrakorporalen Signalgenerator erzeugte Anregungssignal drahtlos in einen intrakorporal angeordneten und mittels zumindest der RF-Spule (18) gebildeten Schwingkreis der induktiven Detektions-Vorrichtung (16) einkoppelbar ist.

2. NMR-Messgerät (100) nach Anspruch 1, **dadurch gekennzeichnet, dass** die Magnet-Vorrichtung (12) zumindest einen Elektromagnet und/oder zumindest einen Permanentmagnet (14) aufweist.

3. NMR-Messgerät (100) nach einem der vorhergehenden Ansprüche, **dadurch gekennzeichnet, dass** die Magnet-Vorrichtung (12) eine Mehrzahl von Magneten umfasst, insbesondere ein Array von Elektromagneten und/oder ein Array von Permanentmagneten (14).

4. NMR-Messgerät (100) nach einem der vorhergehenden Ansprüche, insbesondere nach Anspruch 1, **dadurch gekennzeichnet, dass** die induktive Detektions-Vorrichtung (16) ein Array von RF-Spulen (18), insbesondere ein Array von planaren RF-Spulen, aufweist.

5. NMR-Messgerät (100) nach einem der vorhergehenden Ansprüche, **dadurch gekennzeichnet, dass** die Magnet-Vorrichtung (12) und die induktive Detektions-Vorrichtung (16) zueinander eine vorgegebene, insbesondere feste, Anordnung aufweisen.

6. NMR-Messgerät (100) nach einem der vorhergehenden Ansprüche, **dadurch gekennzeichnet, dass** die Sensorvorrichtung (10) induktiv mit einer extrakorporalen Energieversorgungsvorrichtung (202) koppelbar ist, sodass in der Sensorvorrichtung (10) eine Stromversorgung zum Betrieb der Sensorvorrichtung (10) induzierbar ist.

7. NMR-Messgerät (100) nach einem der vorhergehenden Ansprüche, **dadurch gekennzeichnet, dass** das NMR-Messgerät (100) eine intrakorporal anordenbare Energieversorungsvorrichtung umfasst, wobei die Sensorvorrichtung (10) elektrisch mit der Energieversorgungsvorrichtung gekoppelt ist, die dazu eingerichtet ist, eine Stromversorgung zum Betrieb der Sensorvorrichtung (10) bereitzustellen.

8. NMR-Messgerät (100) nach einem der vorhergehenden Ansprüche, **dadurch gekennzeichnet, dass** die elektrische Schaltung (20) eine Speichervorrichtung (38) zur zumindest vorübergehenden Datenspeicherung aufweist.

9. NMR-Messgerät (100) nach einem der vorhergehenden Ansprüche, **gekennzeichnet durch** eine Ausgabevorrichtung (24), die der Sensorvorrichtung (10) zugehörig realisiert ist und/oder durch eine Ausgabevorrichtung (210), die extrakorporal anordenbar und dem NMR- Messgerät (100) zugehörig und mit der Sensorvorrichtung (10) über eine Datenkommunikationsschnittstelle (204, 26) verbindbar realisiert ist.

10. Verfahren zur Verwendung eines NMR- Messgeräts (100) nach einem der Ansprüche 1-9 zur intrakorporalen NMR-spektroskopischen Untersuchung eines menschlichen oder tierischen Körpers (500) wobei die Sensorvorrichtung (10) intrakorporal angeordnet und der andere Teil (200) extrakorporal angeordnet ist.

## Claims

1. NMR measuring apparatus (100) comprising at least one sensor device (10) specifically configured for intracorporeal arrangement in a human or animal body (500) and comprising at least one other non-biocompatible and non-biofunctional part (200) configured for extracorporeal arrangement,
the sensor device (10) at least comprising:
• a magnet device (12) for creating a homogenous magnetic field B0,
• an inductive detection device (16) for transmitting a nuclear magnetic resonance excitation signal and for receiving a nuclear magnetic resonance signal, for which purpose the inductive detection device (16) comprises at least one RF coil (18), and
• an application-specific electric circuit (20) at least for preprocessing nuclear magnetic resonance signals received by means of the inductive detection device (16), and
• a biocompatible and biofunctional housing (22),
the other part (200) comprising a signal generator having a circuit for creating the excitation signal,
the NMR measuring apparatus (100) being configured for intracorporeal measurement of NMR spectra by means of the sensor device (10),
the inductive detection device (16) of the sensor device (10) being inductively couplable to the signal generator of the NMR measuring apparatus (100) for the purpose of input coupling the excitation signal when said signal generator is arranged extracorporeally,
and the NMR measuring apparatus (100) having a means suitable for inductive coupling, by means of which the excitation signal created by the extracorporeal signal generator is wirelessly input-couplable into an intracorporeally arranged resonant circuit of the inductive detection device (16) formed by means of at least the RF coil (18).

2. NMR measuring apparatus (100) according to Claim 1, **characterized in that** the magnet device (12) comprises at least one electromagnet and/or at least one permanent magnet (14).

3. NMR measuring apparatus (100) according to either of the preceding claims, **characterized in that** the magnet device (12) comprises a plurality of magnets, in particular an array of electromagnets and/or an array of permanent magnets (14).

4. NMR measuring apparatus (100) according to any of the preceding claims, in particular according to Claim 1, **characterized in that** the inductive detection device (16) comprises an array of RF coils (18), in particular an array of planar RF coils.

5. NMR measuring apparatus (100) according to any of the preceding claims, **characterized in that** the magnet device (12) and the inductive detection device (16) have a specified, in particular fixed, arrangement relative to one another.

6. NMR measuring apparatus (100) according to any of the preceding claims, **characterized in that** the sensor device (10) is inductively couplable to an extracorporeal power supply device (202) such that a current supply for operating the sensor device (10) is inducible in the sensor device (10).

7. NMR measuring apparatus (100) according to any of the preceding claims, **characterized in that** the NMR measuring apparatus (100) comprises an intracorporeally arrangeable power supply device, the sensor device (10) being electrically coupled to the power supply device which is configured to provide a current supply for operating the sensor device (10).

8. NMR measuring apparatus (100) according to any of the preceding claims, **characterized in that** the electric circuit (20) comprises a memory device (38) for at least temporary data storage.

9. NMR measuring apparatus (100) according to any of the preceding claims, **characterized by** an output device (24) realized in a manner associated with the sensor device (10) and/or by an output device (210) which is extracorporeally arrangeable and realized in a manner associated with the NMR measuring apparatus (100) and connectable to the sensor device (10) by way of a data communications interface (204, 26).

10. Method for using an NMR measuring apparatus (100) according to any of Claims 1-9 for the intracorporeal NMR-spectroscopic examination of a human or animal body (500), the sensor device (10) being arranged intracorporeally and the other part (200) being arranged extracorporeally.

## Revendications

1. Appareil de mesure à résonance magnétique nucléaire (100), comprenant au moins un dispositif capteur (10) spécialement conçu pour être disposé de manière intracorporelle à l'intérieur du corps d'un humain ou d'un animal (500), et comprenant au moins une autre partie (200) non biocompatible et non biofonctionnelle, qui est conçue pour être disposée de manière extracorporelle,
le dispositif capteur (10) comportant au moins :
• un dispositif magnétique (12) destiné à la génération d'un champ magnétique B0 homogène,
• un dispositif de détection inductif (16) destiné à l'émission d'un signal d'excitation de résonance magnétique nucléaire et à la réception d'un signal de résonance magnétique nucléaire, le dispositif de détection inductif (16) comportant à cet effet au moins une bobine RF (18), et
• un circuit électrique à application spécifique (20) destiné au moins au prétrai-tement de signaux de résonance de spin nucléaire reçus par le dispositif de détection inductif (16), et
• un boîtier biocompatible et biofonctionnel (22),
l'autre partie (200) comportant un générateur de signaux équipé d'un circuit destiné à la génération du signal d'excitation,
l'appareil de mesure à résonance magnétique nucléaire (100) étant conçu pour la mesure intracorporelle, au moyen du dispositif capteur (10), de spectres de résonance magnétique nucléaire,
le dispositif de détection inductif (16) du dispositif capteur (10) pouvant être couplé de manière inductive au générateur de signaux de l'appareil de mesure à résonance magnétique nucléaire (100) pour le couplage du signal d'excitation, lorsque celui-ci est disposé de manière extracorporelle,
l'appareil de mesure à résonance magnétique nucléaire (100) comportant des moyens appropriés pour le couplage inductif, à l'aide desquels le signal d'excitation généré par le générateur de signaux extracorporel peut être couplé sans fil dans un circuit oscillant du dispositif de détection inductif (16), disposé de manière intracorporelle et formé au moyen d'au moins la bobine RF (18).

2. Appareil de mesure à résonance magnétique nucléaire (100) selon la revendication 1, **caractérisé en ce que** le dispositif magnétique (12) comporte au moins un électroaimant et/ou au moins un aimant permanent (14).

3. Appareil de mesure à résonance magnétique nucléaire (100) selon l'une des revendications précédentes, **caractérisé en ce que** le dispositif magnétique (12) comprend une pluralité d'aimants, notamment un réseau d'électroaimants et/ou un réseau d'aimants permanents (14).

4. Appareil de mesure à résonance magnétique nucléaire (100) selon l'une des revendications précédentes, notamment selon la revendication 1, **caractérisé en ce que** le dispositif de détection inductif (16) comporte un réseau de bobines RF (18), notamment un réseau de bobines RF planes.

5. Appareil de mesure à résonance magnétique nucléaire (100) selon l'une des revendications précédentes, **caractérisé en ce que** le dispositif magnétique (12) et le dispositif de détection inductif (16) présentent entre eux un agencement prédéfini, notamment fixe.

6. Appareil de mesure à résonance magnétique nucléaire (100) selon l'une des revendications précédentes, **caractérisé en ce que** le dispositif capteur (10) peut être couplé par induction à un dispositif d'alimentation en énergie extracorporel (202), de sorte qu'une alimentation électrique peut être induite dans le dispositif capteur (10) pour le fonctionnement du dispositif capteur (10).

7. Appareil de mesure à résonance magnétique nucléaire (100) selon l'une des revendications précédentes, **caractérisé en ce que** l'appareil de mesure à résonance magnétique nucléaire (100) comprend un dispositif d'alimentation en énergie pouvant être disposé de manière intracorporelle, le dispositif capteur (10) étant couplé électriquement au dispositif d'alimentation en énergie, qui est conçu pour fournir une alimentation en énergie destinée au fonctionnement du dispositif capteur (10).

8. Appareil de mesure à résonance magnétique nucléaire (100) selon l'une des revendications précédentes, **caractérisé en ce que** le circuit électrique (20) comporte un dispositif de mémoire (38) destiné au stockage au moins temporaire de données.

9. Appareil de mesure à résonance magnétique nucléaire (100) selon l'une des revendications précédentes, **caractérisé par** un dispositif de sortie (24) qui est réalisé de manière à faire partie du dispositif de détection (10) et/ou par un dispositif de sortie (210) qui peut être disposé de manière extracorporelle et qui est réalisé de manière à faire partie de l'appareil de mesure à résonance magnétique nucléaire (100) et qui peut être relié au dispositif capteur (10) par l'intermédiaire d'une interface de communication de données (204, 26).

10. Procédé d'utilisation d'un appareil de mesure à résonance magnétique nucléaire (100) selon l'une des revendications 1-9 pour l'examen spectroscopique par résonance magnétique nucléaire intracorporel du corps d'un humain ou d'un animal (500), le dispositif capteur (10) étant disposé de manière intracorporelle et l'autre partie (200) étant disposée de manière extracorporelle.
